# EUROPEAN PATENT SPECIFICATION

(11) **EP 0 457 438 B1**
(45) Date of publication and mention of the grant of the patent: **12.04.1995**
(21) Application number: 91303467.4
(22) Date of filing: 18.04.1991
(51) Int. Cl.: A61K 47/48, A61K 49/00

(54) **Conjugate compounds containing aza-macrocycles, and processes for their preparation**
Aza-Makrozyklen enthaltende Konjugate und Verfahren zu deren Herstellung
Composés conjugués contenant des macrocycles aza, et leurs procédés de préparation

(30) Priority: 18.04.1990 GB 9008724
(43) Date of publication of application: 21.11.1991
(73) Proprietor: CELLTECH THERAPEUTICS LIMITED, Slough, Berkshire SL1 4EN (GB)
(72) Inventor: Parker, David, Durham, DH1 4DG (GB); Millican, Thomas Andrew, Maidenhead, Berkshire, SL6 ODY (GB); Beeley, Nigel Robert Arnold, Thame, Oxfordshire, OX9 3LQ (GB)
(74) Representative: Hallybone, Huw George

(56) References cited:
- EP-A- 0 382 582
- EP-A- 0 404 605
- WO-A-89/01475
- WO-A-89/01476
- J. CHEM. SOC. CHEM. COMMUN., 1st December 1990, pages 1739-1791, London, GB; C.J. BROAN et al.: "Synthesis and complexation behaviour of an effective octadentate complexone 1,4,7,10-tetraazacyclododecane-1,4,7,10-tetrakis[methylene(methylphosphinic acid)]"
- J. CHEM. SOC. CHEM. COMMON. 1st December 1990, pages 1738-1739, London, GB; C.J. BROAN et al.: "Synthesis and complex stability of parent and C-functionalised derivatives of 1,4,7-triazacyclononane-1,4,7-tris[methylene(methylphosphinic acid)]: an effective new complexing agent"
- BULLETIN OF THE ACADEMY OF SCIENCES OF HET USSR DIVISION OF CHEMICAL SCIENCE,vol. 33, no. 4, part 1, April 1984, pages 769-777, Plenum Publishing Corp., NewYork, US; M.I. KABACHNIK et al.: "Synthesis and study of a new complexone - N,N',N"-tris-(dihydroxyphosphorylmethyl)-1,4,7-triazacyclononane"
- BULLETIN OF THE ACADEMY OF SCIENCES OF THE USSR DIVISION OF CHEMICAL SCIENCE,vol. 33, no. 4, part 1, April 1984, pages 777-782, Plenum Publishing Corp., New York, US; I.M. KABACHNIK et al.: "Synthesis and acid-base and complex-forming properties of 1,4,7,10-tetrakis (dihydroxyphosphorylmethyl)-1,4,7,10-tetraazacyclododecane"
- CHEMICAL ABSTRACTS, vol. 100, 1984, page 420, abstract no. 198535c, Columbus,Ohio, US; K.B. YATSIMIRSKII et al.: "Reaction of 2,4-dinitrophenolates ofalkali metals with N,N',N"-tris(diphenylphosphinylmethyl)-1,4,7-triazacyclononanes" & ZH. NEORG. KHIM. 1984, 29(4), 884-7

## Description

### Field of the Invention

This invention relates to conjugate compounds containing the functionalised aza macrocycles and metal complexes thereof and to processes for their preparation, useful in diagnosis and therapy.

### Background to the Invention

The attachment of metal ions to proteins, peptides and other, smaller molecules is a fast expanding technology, which has numerous proven and potential applications in research, in industry and, particularly, in medicine.

In recent years, much of the impetus behind the development of this technology has been the ability to link metal ions to antibodies, especially monoclonal antibodies. Such metal labelled antibodies have found a widespread use, especially in medicine, where they have been employed, for example, to target the metal ion to a specific tissue type, both in vitro and in vivo. Thus, metal labelled antibodies have applications in locating specific tissue types (e.g. employing computer-aided tomographic techniques where the metal ion is in some way detectable) and in the treatment of cell disorders (e.g. treating mammalian tumours where the metal ion is a cytotoxic radionuclide).

Conventionally, attachment of the metal ion to a protein such as an antibody has been achieved by complexation by an acyclic chelate such as a substituted diethylenetriaminepentaacetic acid [Gansow O. A. et al, Inorg. Chem., (1986), 25, 2772] or ethylenediaminetetraacetic acid [Meares, C. F. et al, Acc. Chem. Res., (1984), 17, 202] covalently linked to the antibody. Such acyclic complexes however tend to be unstable in vivo either as a result of acid-catalysed decomplexation or competitive chelate binding by Ca²⁺ or Zn²⁺ in serum, or as a result of competition from transferrin [Moerlein, S. M. et al, Int. J. Nuc. Med. Biol., (1981) 8, 277]. The lack of stability can result in uncomplexed metal atoms in the body which have a cytotoxic effect on healthy tissue (e.g. bone marrow) or which markedly reduce the signal-to-noise ratio of an imaging technique.

A possible alternative to the use of acyclic chelates in the labelling of antibodies is the use of macrocyclic ligands, which has been suggested by a number of workers [Gansow O. A. et al. Am. Chem. Soc. Symp. Ser., (1984), 241, 215; UK Patent Specification Publication No. 2122641; International Patent Specifications Nos. WO89/01475 and WO89/01476 and European Patent Specification No. 305320; and Moi M. K. et al, Anal. Biochem., (1985), 148, 249-253].

We have now found a new class of functionalised aza macrocycles, members of which are able to form kinetically inert complexes with metal ions. The macrocycles are particularly useful for attachment to proteins, especially antibodies, to provide conjugate compounds capable of binding metals with good association rates to give complexes which are advantageously stable in vivo and which possess an advantageous biodistribution profile.

Thus, according to one aspect of the present invention we provide a conjugate compound comprising a compound of general formula (1); 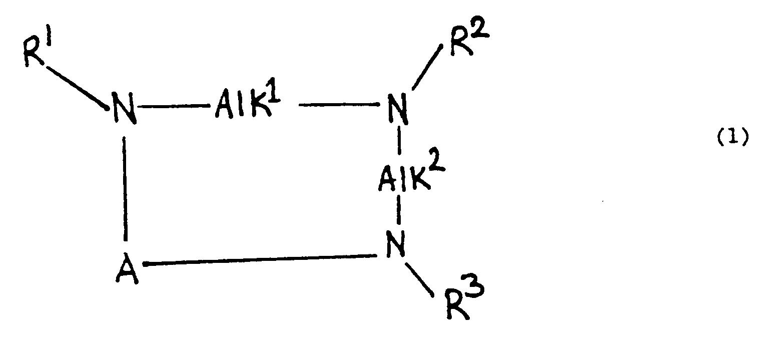
wherein
A is a group -Alk³- or -Alk³N(R⁴)Alk⁴-;
Alk¹, Alk², Alk³ and Alk⁴ which may be the same or different is each a C₁₋₄alkylene chain optionally substituted by one or more C₁₋₆alkyl groups;
and R¹, R², R³ and R⁴, which may be the same or different, is each a hydrogen atom or a group -AlkR⁵ where Alk is an optionally substituted straight or branched C₁₋₆ alkyl group and R⁵ is a hydrogen atom or a -CO₂H, -CONR⁶R⁷ (where R⁶ and R⁷, which may be the same or different is each a hydrogen atom or an alkyl group) or -P(X¹)(X²R⁸)L group where X¹ and X² is each an oxygen or sulphur atom, R⁸ is a hydrogen atom or an alkyl group and L is an aliphatic, aromatic, or heteroaromatic group or a linker group with the proviso that at least one of R¹, R², R³ and R⁴ is a group AlkP(X¹)(X²R⁸)L where L is a linker group; and protected derivatives and metal complexes and/or salts thereof, coupled to a protein, peptide or carbohydrate.

It will be appreciated that formula (1) [and, where appropriate, the following formulae herein], is intended to cover all stereoisomers of the compounds concerned, including mixtures thereof.

The compound of formula (1) may be coupled through any thiol, amino, carboxyl, hydoxyl, aldehyde, aromatic or heteroaromatic group present in the protein, peptide or carbohydrate.

In a preferred aspect of the invention, we provide a conjugate compound which comprises a compound of formula (1) or a metal complex and/or salt thereof, coupled to an antibody.

It is to be understood that conjugate compounds according to the invention may contain more than one molecule of a compound of formula (1) coupled to any one protein, peptide or carbohydrate molecule.

Alk¹, Alk², Alk³ and Alk⁴ in the compounds of formula (1) may each be a chain -CH₂-, -(CH₂)₂-, -(CH₂)₃- or -(CH₂)₄-, optionally substituted by one or more C₁₋₆ alkyl, e.g. methyl or ethyl groups. Examples of substituted alkyl groups represented by Alk¹, Alk², Alk³ and Alk⁴ include -CH₂CH(CH₃)- or -CH₂C(CH₂)₂-.

When the group L in compounds of formula (1) is an aliphatic group it may be for example an optionally substituted straight or branched chain alkyl, alkenyl, alkynyl, alkoxy or alkylthio group, optionally interrupted by one or more heteroatoms, or a cycloalkyl or cycloalkenyl group. When L is an aromatic group it may be for example an aryl or aralkyl group. Heteroaromatic groups represented by L include heteroaryl and heteroaralkyl groups.

Thus, for example, L may be an optionally substituted C₁₋₁₀alkyl (e.g. C₁₋₆ alkyl such as methyl, ethyl, n-propyl, i-propyl, n-butyl, s-butyl or t-butyl) C₂₋₁₀alkenyl (e.g. C₂₋₆ alkenyl such as ethene, propene, 1-butene, 2-butene, or 2-methylpropene), C₂₋₁₀alkynyl (e.g. C₂₋₆ alkynyl such as ethyne, propyne, 1-butyne, or 2-butyne) C₁₋₁₀alkoxy (e.g. C₁₋₆alkoxy such as methoxy, ethoxy, n-propoxy, i-proproxy, n-butoxy, s-butoxy, or t-butoxy) or C₁₋₁₀alkylthio (e.g. C₁₋₆alkylthio such as methylthio, ethylthio, n-propylthio, i-propylthio, n-butylthio, s-butylthio, or t-butylthio) group optionally interrupted by one or more heteroatoms selected from -O-, -S- or NR⁶ (where R⁶ is a hydrogen atom or a C₁₋₆ alkyl group), for example an alkoxyalkyl (e.g. methoxymethyl) alkylthioalkyl (e.g. methylthiomethyl) or alkoxyalkoxy or alkylthioalkoxy (e.g. methoxymethoxy or methylthiomethoxy) group; or a C₃₋₈ cycloalkyl (e.g. cyclopropyl, cyclobutyl, cyclopentyl, or cyclohexyl) or C₄₋₈ cycloalkenyl (e.g. cyclobutene, cyclopentene, cyclohexene, or cyclohexadiene) group.

When L is an aryl group it may be for example an optionally substituted C₆₋₁₂aryl group such as an optionally substituted phenyl or naphthyl group.

When L is an aralkyl group it may be for example an optionally substituted C₆₋₁₂arC₁₋₆alkyl group for example a phenC₁₋₆alkyl group such as benzyl or phenethyl.

When L is a heteroaryl group it may be for example an optionally substituted C₄₋₁₀heteroaryl group containing one or more heteroatoms selected from -O-, -NH- or -S-, for example a pyridyl, furanyl or thienyl group.

When L is a heteroaralkyl group it may be for example an optionally substituted C₄₋₁₀heteroarC₁₋₆alkyl group containing one or more heteroatoms selected from -O-, -NH- or -S- for example a thienylC₁₋₆alkyl (e.g. thienylmethyl) or pyridylC₁₋₆alkyl (e.g. pyridylmethyl) group.

Optional substituents which may be present on alkyl, alkoxy, aryl, aralkyl, heteroaryl or heteroaralkyl groups present in the group L in compounds of formula (1) include one or more halogen atoms e.g. chlorine, bromine, fluorine or iodine atoms, or one or more groups selected from hydroxyl, C₁₋₆ alkyl e.g. methyl or ethyl, C₁₋₆ alkoxy, e.g. methoxy or ethoxy, C₁₋₆alkylthio, e.g. methylthio, amino (-NH₂), substituted amino, e.g. NR⁷R⁸ where R⁷ is a hydrogen atom or a C₁₋₆alkyl group and R⁸ is a C₁₋₆ alkyl group, (such as methylamino or dimethylamino), nitro, cyano, carboxyl, -CONR⁶R⁷ (e.g. -CONH₂), -SO₂NR⁶R⁷ (e.g. SO₂NH₂) or C₃₋₈cycloalkyl (e.g. cyclopropyl, cyclobutyl, cyclopentyl or cyclohexyl) groups.

In the compounds of formula (1), it will be appreciated that the nature of the linker group represented by L may be varied widely without substantially affecting the usefulness of the compounds. Thus the linker group L may be a group of formula -L¹(Z)w where L¹ is an optionally substituted polyvalent, especially bivalent, radical of an aliphatic, aromatic, heteroaromatic or araliphatic compound, Z is the residue of a reactive functional group and w is zero or an integer 1.

When L¹ is an aliphatic group, it may be for example an optionally substituted aliphatic hydrocarbyl chain, optionally interrupted by one or more heteroatoms selected from -O- or -S- or by one or more -N(R⁹)- (where R⁹ is a hydrogen atom or a C₁₋₆alkyl group), -CON(R⁹)-, -N(R⁹)CO-, cycloaliphatic, aromatic, or heteroaromatic groups.

In the above definition, and in the same context whenever it appears below, the term "interrupted by" as applied to cycloaliphatic or aromatic groups is to be understood to also mean that these particular groups may additionally be present linked to the terminal carbon atom of the hydrocarbyl chain represented by L¹, at the opposite end of the chain to the carbon atom attached to the macrocycle.

Thus, for example, L¹ may be an optionally substituted straight or branched C₁₋₂₀alkylene, C₂₋₂₀alkenylene, or C₂₋₂₀alkynylene chain, optionally interrupted by one or more -O- or -S- atoms or C₅₋₈cycloalkylene (e.g. cyclopentylene or cyclohexylene), C₆₋₁₂aromatic (e.g. phenylene or substituted phenylene), C₅₋₁₀heteroaromatic (e.g. furanyl, pyridyl), -N(R⁹)-, -CON(R⁹)- or -N(R⁹)CO- groups.

When L¹ is an aromatic group it may be an aryl group, for example a C₆₋₁₂aryl group such as a phenyl group.

When L¹ is a heteroaromatic group it may be for example an optionally substituted heteroaryl or heteroaralkyl group e.g. a C₄₋₁₀heteroaryl or C₄₋₁₀heteroar C₁₋₆alkyl group containing one or more heteroatoms selected from -O-, -NH- or -S-, for example a pyridyl, pyridylC₁₋₆alkyl, e.g. pyridylmethyl, furanyl, furanylC₁₋₆alkyl, e.g. furanylmethyl, thienyl or thienylc₁₋₆alkyl, e.g. thienylmethyl.

Examples of substituents which may be present on the group L¹ include one or more halogen atoms, e.g. fluorine, chlorine, bromine, or iodine atoms or one or more groups selected from C₁₋₆ alkyl (e.g. methyl or ethyl), C₁₋₆alkoxy (e.g. methoxy or ethoxy), C₁₋₆alkylthio e.g. methylthio, hydroxy, nitro, -N(R¹⁰)(R¹¹), [where R¹⁰ is a hydrogen atom or a C₁₋₆alkyl group and R¹¹ is a C₁₋₆alkyl group; e.g. -NHCH₃ or -N(CH₃)₂], or substituted amido, e.g. a group of formula -(CH₂)_{d}CON(R¹²)(R¹³) [where d is zero or an integer 1 to 4 inclusive, R¹² is a hydrogen atom or a C₁₋₆alkyl group, e.g. methyl and R¹³ is an optionally substituted C₁₋₆alkyl group].

Substituted alkyl groups represented by R¹³ include for example C₁₋₆alkyl groups substituted by one or more halogen atoms, or nitro, amino or hydroxy groups.

The residue of a reactive functional group represented by Z may in general be the residue of any group capable of reacting with a thiol, amino, carboxyl, hydroxyl, aldehyde, aromatic or heteroaromatic group. Aromatic groups include, for example, phenolic groups. Heteroaromatic groups include for example imidazolyl groups.

In particular, Z may be for example -S-, -NH-, -NHN-, -N(CH₃)N=, -NHCONHN=, -NHCSNHN=, -N(Ph)N=, (where Ph is optionally substituted phenyl), -NC(O)-, -NC(S)-, -CO-, a vinyl group of formula -Het¹- C(Het²)CH₂ (where Het¹ and Het², which may be the same or different, is each a nitrogen containing heterocyclic group, e.g. a pyridyl group or Het¹ is a nitrogen containing heterocyclic group and Het² is a hydrogen atom), for example a vinyl pyridyl group of formula 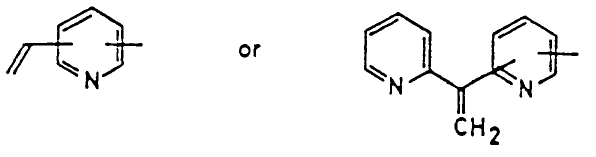
especially 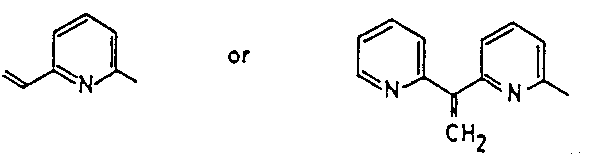
an imide, or a dione of formula 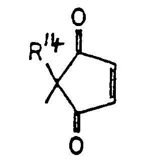
(where R¹⁴ is a C₁₋₄alkyl e.g. methyl group).

In the compounds of formula (1) alkyl groups represented by R⁶, R⁷ or R⁸ may be straight or branched chain groups and may be for example C₁₋₆ alkyl groups such as methyl or ethyl groups.

The group CONR⁶R⁷ when present in compounds of formula (1) may be for example -CONH₂, -CONHCH₃, -CON(CH₃)₂, -CONHCH₂CH₃ or -CON(CH₂CH₃)₂.

Alk in the compounds of formula (1) may be for example a methyl, ethyl, n-propyl, i-propyl, n-butyl, s-butyl or t-butyl group. Such groups may be substituted, for example, by one or more halogen atoms, e.g. fluorine or chlorine atoms.

Metal complexes of the compounds of formula (1) in which A is -Alk³N(R⁴)Alk⁴- include complexes wherein the metal is di- or tripositive and has a coordination number 6 or greater, especially 8. Examples of such metals include manganese (Mn), iron (Fe), indium (In), copper (Cu), lead (Pb), bismuth (Bi), yttrium (Y), terbium (Tb), gallium (Ga), gadolinium (Gd), scandium (Sc), other transition metals with atomic numbers 21-29, 42, 43, 44 or 75, and other lanthanides with atomic numbers 57-70. In, Y, Ga, Tb, Gd, and Sc are preferred, particularly In, Y, Gd, and Ga. The metal may be a radioactive isotope, for example ⁹⁰Y.

Metal complexes of the compounds of formula (1) in which A is -Alk³- include complexes wherein the metal is di- or tri-positive and has a coordination number from 2 up to 6, especially 6. Examples of such metal(s) include indium (In), copper (Cu), lead (Pb), bismuth (Bi), colbalt (Co), gadolinium (Gd) and gallium (Ga). In, Ga, Gd, Co and Cu are preferred, particularly In, Gd and Ga. In general the metal is preferably a radioactive isotope. Indium, especially ¹¹¹In, is particularly preferred.

In general, optimum binding of the metal to the compounds of formula (1) may be achieved by selection of the ring size and where appropriate by adjusting the potential coordination number by choice of the group R¹, R², R³ or R⁴.

Salts of the compounds of formula (1) or metal complexes thereof include salts with inorganic or organic bases, for example alkali metal or alkaline earth metal salts such as lithium, sodium, potassium, magnesium or calcium salts; amine salts such as those from primary, secondary or tertiary amines, for example ethanolamine, diethanolamine, morpholine, glucamine, N-methylglucamine, or N,N-dimethylglucamine salts; and amino acid salts such as lysine, arginine and ornithine salts. Pharmaceutically acceptable salts are particularly preferred.

A particularly useful group of conjugate compounds according to the invention is that wherein the compound of formula (1) A is -Alk³- or -Alk³N(R⁴)Alk⁴- and Alk¹, Alk², Alk³ and Alk⁴ is each a chain -(CH₂)₂-.

One group of compounds of formula (1) has the formula (1a): 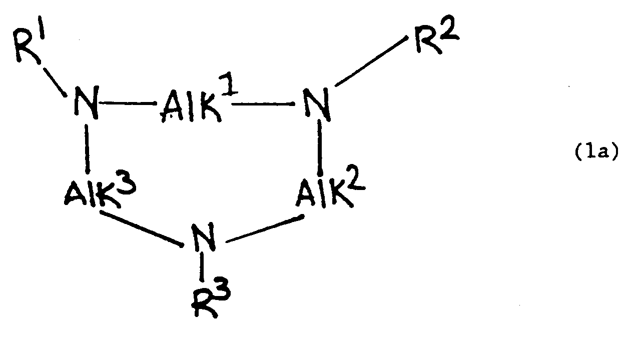
wherein R¹, R², R³, Alk¹, Alk² and Alk³ are as defined for formula (1) with the proviso that at least one of R¹, R² and R³ is a group AlkP(X¹)(X²R⁸)L where L is a linker group; and metal complexes and/or salts thereof.

Indium complexes of the compounds of formula (1a) are particularly preferred.

Particularly important compounds of formula (1a) are those of formula (1b) 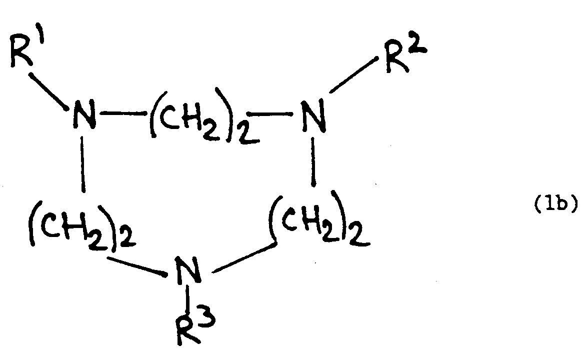
wherein R¹, R² and R³ are as defined for formula (1a) and metal complexes and/or salts thereof.

Indium complexes of the compounds of formula (1a) are particularly preferred.

Another group of compounds of formula (1) has the formula (1c): 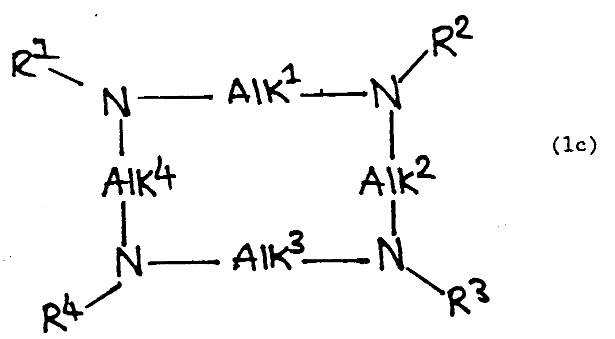
wherein R¹, R², R³, R⁴, Alk¹, Alk², Alk³ and Alk⁴ are as defined for formula (1) with the proviso that at least one of R¹, R², R³ and R⁴ is a group AlkP(X¹)(X²R⁸)L where L is a linker group; and protected derivatives and metal complexes and/or salts thereof.

Yttrium and gadolinium complexes of the compounds of formula (1c) are particularly preferred.

An important group of compounds of formula (1c) are those of formula (1d) 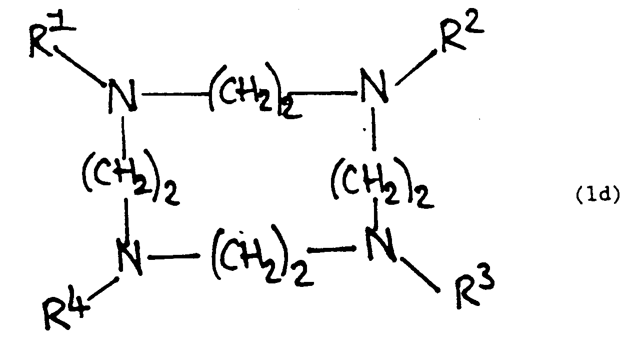
wherein R¹, R², R³ and R⁴ are as defined for formula (1c); and metal complexes and/or salts thereof.

Yttrium and gadolinium complexes of the compounds of formulae (1d) are particularly preferred.

In general, in the compounds of the various formulae described above, the group AlkP(X¹)(X²R⁸)L when present is preferably a group AlkP(O)(OR⁸)L, for example a group AlkP(O)(OH)L, such as -CH₂P(O)(OH)L. Particular instances of groups of this latter type are those of formula -CH₂P(O)(OH)L where L is C₁₋₆alkyl e.g. methyl or -L¹-(Z)w.

The groups R¹, R², R³ and R⁴ in the various formulae described above are preferably groups -AlkR⁵ where Alk is an optionally substituted straight or branched C₁₋₆alkyl group, and is particularly a methylene group, and R⁵ is a CO₂H, -CONR⁶R⁷ or -P(X¹)(X²R⁸)L group, particularly a group -P(O)(OH)L.

The metal complexes of the conjugate compounds have a diagnostic use as imaging agents, for example as contrast agents, in vitro and in vivo. The compounds of formula (1) and the metal complexes and/or salts thereof are also cytotoxic agents and may be used in the treatment of abnormal cell disorders, for example in the treatment of tumours. For use as diagnostic and/or therapeutic agents, conjugates may be employed using conventional methods, (e.g. for formulation and presentation) already in use for metal complexing agents.

Particularly useful conjugate compounds according to the invention are those comprising a compound of formula (1b) or formula (1d) or a metal complex and/or salt thereof, coupled to an antibody. The indium, yttrium and gadolinium complexes of these conjugates are especially important.

The antibody in conjugates according to the invention may in general belong to any immunoglobulin class. Thus for example it may be an immunoglobulin M antibody or, in particular, an immunoglobulin G antibody. The antibody molecule may be of animal, for example mammalian origin, and may be for example of murine, rat or human origin. It may be a natural antibody or a fragment thereof, or, if desired, a recombinant antibody or antibody fragment i.e. an antibody molecule or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework region of a second, different antibody (as described in European Patent Specification No. 239400); (2) recombinant antibodies or fragments wherein non-Fv sequences have been substituted by non-Fv sequences from other, different antibodies (as described in European Patent Specification Nos. 171496, 173494 and 194276); or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is in the place of another amino acid residue present in the natural immunoglobulin (as described in International Patent Applications Nos. WO89/01974 and WO89/01782 respectively).

The antibody may be of polyclonal or, preferably, monoclonal origin. It may be specific for any number of antigenic determinants, but is preferably specific for one. The antigenic determinants may be any hapten or antigenic determinant associated with any antigen. Particular antigens include those associated with animals, e.g. humans, [for example normal animal tissue or organ cell-associated antigens, tumour cell associated antigens (for example oncofetal antigens such as carcinoembryonic antigen or alphafetoprotein, placental antigens such as chorionic gonadotropin and placental alkaline phsophatase, and prostate antigens such as prostatic acid phsophatase and prostate specific antigen) and antigens associated with components of body fluids such as fibrin or platelets], viruses, bacteria and fungi.

In a preferred aspect the antibody may be capable of recognising and binding a tumour cell-associated antigen, particularly one or more epitopes on the TAG-72 antigen associated with human breast and colon tumours. A particularly preferred antibody of this type is the monoclonal antibody B72.3 [Colcher, D. et al Proc. Nat. Acad. Sci. USA (1981), 78 3199] or a fragment thereof, particularly a F(ab')₂ fragment.

The antibody will in general be coupled to the remainder of the conjugate of the invention (i.e. the macrocycle and linker) through any appropriate reactive atom or group, for example a nitrogen or especially, sulphur atom, present in the antibody. It will be appreciated that any one antibody molecule may contain more than one reactive group capable of coupling with the macrocycle and linker.

The conjugate compounds of the invention may be formulated for use in accordance with conventional practice. Thus according to a further aspect of the invention we provide a composition comprising a conjugate compound comprising a compound of formula (1) coupled to a protein, peptide or carbohydrate, or a metal complex and/or salt thereof, together with one or more pharmaceutically acceptable carriers.

Particularly suitable compositions according to the invention are those adapted for parenteral administration, especially by injection or infusion. Suitable formulations of this type include suspensions solutions or emulsions of the conjugate in oily or aqueous vehicles, and may contain formulatory agents such as suspending, stabilising and/or dispersing agents. Alternatively the conjugate may be in powder form for reconstitution with a suitable vehicle, e.g. sterile pyrogen-free water before use. If desired the conjugate may be presented in unit dosage form, and/or together with one or more active ingredients or imaging agents. Suitable formulations of this type include solutions of the conjugate according to the invention in isotonic saline.

The quantities of conjugates of the invention used in formulations according to the invention will vary according to the intended use and, in particular cell target, but may be easily determined in accordance with conventional practice for reagents of this type.

Conjugates of the invention may be prepared by the following processes wherein the groups and symbols A, R¹, R², R³, R⁴, R⁵, Alk, Alk¹, Alk², Alk³ and Alk⁴, are as defined for formula (1) except where stated otherwise. Where a metal complex is desired as a final product, the complexation with a metal atom may be carried out as a final step in the production process, as described below for the complexation of compounds of formulae (1), or alternatively it may be desirable to complex the metal at an earlier stage in the process, providing of course that the requisite macrocycle structure is present. In the following processes, it may be desirable to use starting materials in which functional groups in the linker group are in a protected state, or which contain a precursor of the group, as discussed below.

Metal complexes for use according to the invention may be prepared by reacting a compound of formula (1) or a salt thereof with a metal salt (e.g. a nitrate, halide, such as a chloride, acetate, carbonate or sulphate) or a metal oxide.

The reaction may be performed in an appropriate solvent, for example an aqueous or non-aqueous solvent (e.g. acetonitrile, acetone, propylene carbonate, dimethylformamide or dimethylsulphoxide) at any suitable temperature from 0°C to 100°C such as 10°C to 85°C.

Salts of compounds of formula (1) may be prepared by reacting a compound of formula (1) with a base in an apropriate solvent, for example an aqueous or non-aqueous solvent as described above, at any suitable temperature from 0°C to 100°C.

Compounds of formula (1) in which one or more of R¹, R², R³ and R⁴ is each a group AlkP(X¹)(X²H)L may be prepared by interconversion of a corresponding compound of formula (1) in which one or more of R¹, R², R³ and R⁴ is each a group AlkP(X¹)(X²R⁸)L [where R⁸ is an alkyl group] by treatment with an acid, for example an inorganic acid such as hydrochloric acid at an elevated temperature, for example the reflux temperature.

Compound of formula (1) in which R¹ is a group AlkP(X¹)(X²R⁸)L where L is a linker group, and the remaining groups R², R³ and R⁴ is each a group AlkP(X¹)(X²R⁸)L where R⁸ is an alkyl group, may be prepared by reaction of a compound of formula (2) 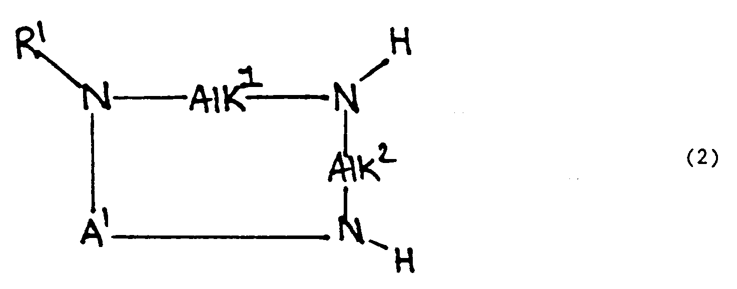
[where R¹ is as just defined and A¹ is -Alk³- or Alk³NHAlk⁴-] with a phosphine L(X¹Alk⁶)(X²R⁸) [where R⁸ is as just defined and Alk⁶ is an alkyl group, for example an ethyl group] in the presence of formaldehyde, paraformaldehyde or an aldehyde RCHO (where R is a C₁₋₅alkyl group).

The reaction may be performed in a solvent, for example an organic solvent such as an ether, e.g. a cyclic ether such as tetrahydrofuran at an elevated temperature e.g. the reflux temperature.

Alternatively, a compound of formula (1) in which R¹ is a group AlkP(X¹)(X²R⁸)L where L is a linker group may be prepared by reaction of compound of formula (2) with a reagent R⁵AlkD where D is a displaceable group such as a halogen, e.g. chlorine, atom or a sulphonyloxy group, e.g. a methanesulphonyloxy group.

The reaction may be performed in a solvent such as water or an organic solvent such as a nitrile, e.g. acetonitrile, or an alcohol, e.g. ethanol, or an amide, e.g. dimethylformamide, in the presence of a base such as an alkali metal carbonate or hydroxide, e.g. sodium, potassium or caesium carbonate, or sodium, potassium or lithium hydroxide, at an elevated temperature e.g. the reflux temperature.

In this reaction, any -CO₂H group present in R⁵AlkD may need to be protected, for example as an ester, e.g. a methyl ester. The acid may be regenerated after the desired reaction is complete, for example by hydrolysis using an acid such as sulphuric acid. Similarly, reactive functional groups in the linker group L may need to be protected. For example amine (NH₂) groups may be protected by acylation, for example as acetylamino or benzoylamino groups. The free amine may be regenerated from such groups by reaction with an acid such as an inorganic acid, e.g. hydrochloric acid, at an elevated temperature.

Compounds of formula (1) may also be prepared by interconversion from other compounds of formula (1). Thus one functional group Z may be exchanged for another and, if desired a linker group L changed to another by appropriate manipulative reactions. For example, a compound of formula (1) where L is a group L²-NHCO-L³-Z (where -L²-NHCO-L³ represents the group L¹) may be prepared by reaction of a corresponding compound wherein L represents L²-NH₂ with a reagent R^{a}O-L³-Z (where R^{a} is for example an imide, such as succinimide, or a substituted phenyl group such as a p-nitrophenyl group) in the presence of a tertiary amine such as diisopropylethylamine or N-methylmorpholine, in a solvent such as dimethylformamide or dimethylsulphoxide.

A conjugate compound according to the invention may be prepared by reaction of a compound of formula (1) or a metal complex thereof [wherein at least one group R¹, R², R³ or R⁴ is a group AlkP(X¹)(X²R⁸)L and L is a group L¹-Z] with a protein, peptide or carbohydrate in a aqueous solvent, for example an inorganic buffer such as a phosphate buffer at an appropriate temperature for example at 0°C-40°C, e.g. 0°C - 10°C.

The protein, peptide or carbohydrate may be obtained using precedures well known in the art. If desired, before the coupling reaction, the protein, peptide or carbohydrate may first be treated to yield appropriate groups for reaction with the compound of formula (1). Thus, for example, the protein, peptide or carbohydrate may be subjected to oxidation, for example periodate oxidation to yield aldehyde groups, or may be treated with a reagent [e.g. Traut's reagent (2-iminoithiolane] using standard procedures to generate free sulphydryl groups in the molecule.

Intermediates of formula (2) may be prepared by reaction of a compound of formula (3) 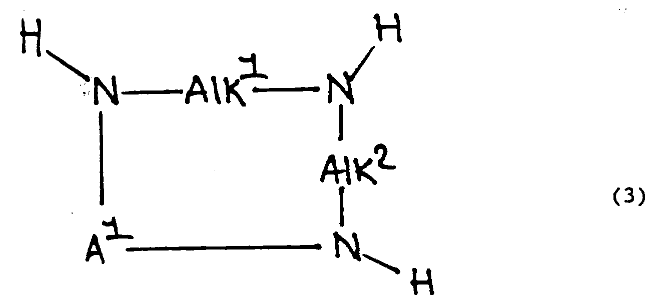
with a compound DAlkP(X¹)(X²R⁸)L in the presence of a base in a suitable solvent at an elevated temperature as just described for the preparation of compounds of formula (1). By varying the molar ratio of the compound of formula (2) and the compound DAlkP(X¹(X²R⁸)L such that the latter is increased relative to the former, (for example from around 2:1 to 1:1 and further) compounds of formula (2) containing more than AlkP(X¹)(X²R⁸)L group as just defined may be prepared.

Intermediates of formula (3) and intermediates DAlkP(X¹)(X²R⁸)L are either known compounds or may be prepared from known starting materials using methods analogous to those used for the preparation of the known compounds for example as described in the following Intermediates and Examples.

The following Intermediates and Example illustrate the invention. The following abbrevations are used: Ph:phenyl; Ms:CH₃SO₂-; Et:ethyl.

### Intermediate 1

### Preparation of HOCH₂P(O)(OH)(CH₂)₃NHCOPh

To a solution of N-benzamido allylamine (7.47g) and hypophosphorus acid (8.66g, 505 solution) in dioxane (100ml) was added t-butylperoxide (0.4g) and the mixture was heated to reflux for 18h. Solvents were removed under reduced pressure and ¹H NMR analysis of the residue revealed that the olefinic resonances had disappeared. The residue was redissolved in dioxane (50ml) and paraformaldehyde (25g) was added and the mixture heated to reflux for 72h. After removal of solvent the residue was chromatographed on silica (eluant 70% CH₂Cl₂, 25% methanol, 5% NH₄OH) to yield the ammonium salt of the title acid as a pale yellow glass: δp (D₂O)+41.1ppm; δ_{c} (D₂O) 170.04 (CONH), 134.0 (C₅H₅CCO); 132.28, 128.98, 127.22 (CH), 59.73 (PCH₂OH, d, J_{CP} 99HZ); 41.01 (CONHCH₂); 25.12 (PCH₂CH₂, d, J_{CP}=81HZ); 22.03 (PCH₂CH₂CH₂NHCO) δ_{H}(D₂O) 7.79 (2H, dd, ortho ArH), 7.57 (4H mult, NHCO +ArH); 3.81 (2H, d, J=6.1Hz, PCH₂OH); 3.71 (2H, t, J=6.9Hz, CH₂NCO), 1.8 (4H, mult., PCH₂CH₂).

### Intermediate 2

### Preparation of HOCH₂P(O)(OEt)(CH₂)₃NHCOPh

To Intermediate 1 (5g) in distilled water (50ml) was added Dowex strong acid ion exchange resin (30g, H⁺ form) and after filtration the filtrate was evaporated under reduced pressure and the residue treated with triethylorthoformate (25ml) and the mixture heated under argon at 90°C for 96h. After removal of HC(OEt)₃ under reduced pressure the residue was chromatographed on silica (CH₂Cl₂ = 5 to 10% methanol gradient) to yield a mixture of the desired alcohol ester and the mixed orthoformate ester. Treatment of this mixture with ethanol (50ml, 1ml concentrated HCl) followed by heating to reflux (36h), evaporation and subsequent chromatographic purification as before yielded the title alcohol ester as a pale yellow oil, (4g). m/e (d.c.i.) 286 (M⁺+1). δp(CDCl₃)53.7ppm δ_{H} (CDCl₃) 7.71 (2H, dd, ortho, CH), 7.25 (3H, mult, arom CH), 6.85 (1H, brt, NHCO), 4.05 (1H, brs OH), 3.81 (2H, dq, CH₂O), 3.70 (1H, br, d, CH₂OH); 3.31 (2H, t HNCH₂), 1.75 (4H, mult., PCH₂CH₂); 1.05 (3H, t, CH₃). δ_{c} (CDCl₃/CD₃CO₂D) 168.56 (CONH) 132.98 (C₅H₅CO); 131.11, 127.82, 126.58 (CH); 56.16 (PCH₂OH, d, J_{CP} = 109Hz); 48.53 (OCH₂CH₃); 39.62 (CONHCH₂); 21.64 (PCH₂, d, J_{CP} = 90Hz); 20.33 (CH₂); 15.37 (CH₃).

### Intermediate 3

### Preparation of MsOCH₂P(O)(OEt)(CH₂)₃NHCOPh

To a suspension of Intermediate 2 (0.57g) in dry tetrahydrofuran (50ml) at 0°C was added triethylamine (1g) and methanesulphonyl chloride (1.14g) under argon. After 2h stirring, ethanol (5ml) was added and the mixture stirred for 20min at 0°C, solvent removed under reduced pressure, and the residue taken up in ethyl acetate (30ml), filtered and evaporated to give a residue which was chromatographed on silica gel (eluant 2 to 5% methanol in CH₂Cl₂) to yield the title mesylate as a colourless oil (390mg) m/e (δ.c.i, CH₂Cl₂) 364 (M⁺+1). δp (CDCl₃) 45.96ppm. δ_{c} (CDCl₃) 168.6 (NHCO); 134.0 (CH₅H₅CCO); 131.4, 128.4, 129.3 (CH); 62.2 (POCH₂), 61.2 (PCH₂OMs, d, J_{PC} = 70Hz); 39.62 (CONHCH₂), 37.6 (OSO₂CH₃); 24.0 (PCH₂CH₂, d, J_{PC} = 100Hz); 21.2 (CH₂), 15.4 (CH₃)

### Intermediate 4

### Preparation of a compound of formula (1d) where R¹ is -CH₂P(O)(OEt)(CH₂)₃NHCOPh and R², R³ and R⁴ is each -H

To a solution of 1, 4, 7, 10-tetrazacyclododecane (0.16g) in dry dimethylformamide (25ml) was added potassium carbonate (0.13g) at 60°C and a solution of Intermediate 3 (0.167g) in dimethylformamide (15ml) over a period of 2h under N₂. After 64h, hplc analysis (CM300) revealed that reaction was not progressing and solvent was removed under reduced pressure. The crude residue was redissolved in dichloromethane (30ml), filtered and evaporated before purification on a CM-300 column to yield the title monoalkylated amine (0.05g) as a pale yellow oil. Rₜ = 8.2min (CM300 hplc). δ_{H} (CDCl₃) 1.30 (3H, t, J=76Hz, OCH₂CH₃), 1.97 (5H, mult, CH₂CH₂N + NH), 2.64-2.94 (20H, mult, CH₂P), 3.55 (2H, dt, CONHCH₂) 4.06 (2H, dq, OCH₂), 7.38-7.47 (3H, mult, aryl CH), 7.93 (2H, dd, orthoCH), 8.55 (1H, t, CONH). m/e (c.i.) 440 (M⁺+1) 394(M⁺-OC₂H₅)

### Intermediate 5

### (a) Preparation of a compound of formula (1d) where R¹ is -CH₂P(O)(OEt)(CH₂)₃NHCOPh and R², R³, and R⁴ is each -CH₂P(O)(OEt)CH₃

To a solution of Intermediate 4 (0.015g) in dry dimethylformamide (1ml) was added potassium carbonate (16mg) and MsOCH₂P(OEt)₂CH₃ (25mg) under N₂. After heating to 80°C for 16h, t.l.c. (Al₂O₃) and hplc analysis (CM300) indicated no further reaction had occurred. After removal of solvent under reduced pressure, the residue was treated with dichloromethane (10ml) filtered and evaporated to yield a residue which was purified by chromatography on alumina (eluant 0 to 2% methanol in CH₂Cl₂) to give the title tetraester as a colourless oil (11mg). Rₜ (CM300, hplc) 4.6min. δ_{H} (CDCl₃) 1.30 (12H, t, J=7.2, CH₃CH₂), 1.49 (9H, d+d+d, PCH₃), 1.80-3.70 (30H, mult., br., CH₂N+CH₂P+CH₂C) 4.05 (8H, dq, OCH₂), 7.39 (3H, mult, arylCH), 7.92 (2H, dd, ortho CH), 8.35 (1H, br, NHCO). m/e (c.i.) 800 (M⁺+1).

### (b) Preparation of a compound of formula (1d) where R¹ is -CH₂P(O)(OH)(CH₂)₃NH₂ and R², R³ and R⁴ is each -CH₂P(O)(OH)CH₃

Hydrolysis of the tetraester of Part (a) (6M hydrochloric acid, 110°C, 48h) afforded after removal of solvent the title amino-tetraacid δ_{H}(CDCl₃) 1.35 (9H, d), 1.55-1.85 (4H, m), 2.6-3.7 (30H, m), 7.35 (2H, d), 8.35 (2H, d).

### Intermediate 6

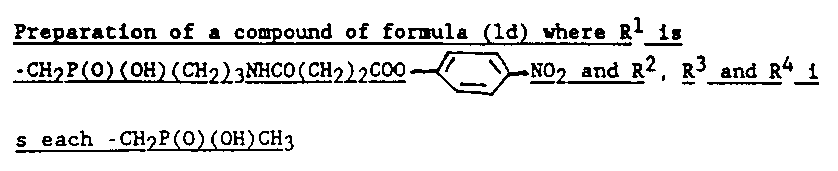

Intermediate 5(b) [27.5mg] was dissolved in dimethylsulphoxide (1ml) with slight heating. N-Methylmorpholine (35»l) was added and the dimethylsulphoxide solution went cloudy and precipitation occurred. Immediately the di-4-nitrophenyl ester of succinic acid (25mg) in dimethylsulphoxide (1.0ml) was added and the reaction mixture heated to 150°C for approximately 1 minute, and then left for a further 90 minutes at 45°C. The reaction mixture was purified by HPLC (Dynamax - Prog MAX4) to yield, after evaporation of solvent under reduced pressure, the title compound as a white powder (10mg). m/e (FAB) 805 (M⁺+1). δ_{H}(CDCl₃) 1.28 (9H, d), 1.5-1.8 (4H, m), 2.75-3.6 (26H, m).

### Intermediate 7

### Preparation of a compound of formula (1b) where R¹ is -CH₂P(O)(OEt)(CH₃)₃NHCOPh and R² and R³ is each -H

Potassium carbonate (0.038g, 0.28mmol) was added to a solution of 1,4,7-triazacylonanine (1) (0.071g, 0.55mmol) in anhydrous dimethylformaide (10cm³) under a nitrogen atmosphere and the mixture was heated to 60°C. A solution of Intermediate 3 [(0.10g) in anhydrous dimethylformamide (10cm³)] was added dropwise over a period of 2h and the mixture stirred for a further 36h at 60°C. The cooled reaction mixture was filtered and solvent removed under reduced pressure to give a pale yellow oil. Purification was afforded by preparative HPLC ('Synchropak' CM300 cation exchange), to afford the title compound (0.03g) as a colourless oil;

HPLC tR 6.8 min observed at λ=254nm ('Synchropak' CM300 cation exchange) with gradient elution, 1.4ml min⁻^{,} A-H₂O, B=1.0M-NH₄OAc, C=MeCN; from t=0 min, 80% A, 0% B, 20% C, to t=5min, 60% A, 20% B, 20% C, to t=10 min, 0% A, 80% B, 20% C;
δ_{H} (400MHz, CDCl₃) 1.56 (3H, t, J 6.8Hz, POCH₂CH₃), 2.26-2.27 (2H, m, PCH₂CH₂), 2.88-3.18 (14H, mult, CH₂N + CH₂PO) 3.22 & 3.25 (2H, ddd, J, 5.2Hz, NCH₂PO) 3.75 & 3.88 (1H+1H, ddt, J 6.4Hz, CH₂NHCO₂PO), 4.33 (2H, d,quart, J 7.2Hz, P.O.CH₂CH₃), 5.49 br (2H, s, 2 x NH CH₂CH₂NH), 7.65-7.71 (3H, m, aromatic C₆H₂H₃), 8.24-8.27 (2H, m, aromatic C₆H₃H₂) and 9.05 (1H, br, t, J 6Hz (NHCOPh), m/z (DCI, NH₃) 398 (M⁺+2), 397 (M⁺+1), 256, 142-

### Intermediate 8

### Preparation of a compound of formula (1b) where R¹ is -CH₃P(O)(OEt)(CH₂)₃NHCOPh and R² and R³ is each -CH₂P(O)(OEt)CH₃

To a stirred solution of Intermediate 7 (0.03g) in dry tetrahydrofuran (20cm³) was added the CH₃P(OEt) (0.03g) followed by formaldehyde (0.01g) under an atomosphere of dry N₂. The resulting mixture was refluxed for 16h, with soxhlet drying using freshly activated 4Å molecular sieves. After cooling the mixture was filtered and the tetrahydrofuran removed under reduced pressure to give a pale yellow oil. The crude title ester was purified via alumina chromatography 13% MeOH in CH₂Cl₂ as eluant) to yield the ester (0.025g) as a colourless oil; HPLC tR 5.1min observed at λ=254mm ("Synchropak ' CM300 cation exchange) with gradient elution, 1.4ml min ⁻¹, A=H₂0, B=1.0M NH₄OAc, C=MeCN; from t=O min, 80%A, 0% B, 20% C, to t=5 min, 60% A, 20% B, 20% C, to t=10 min, 0% A, 80% B, 20% C;
δ_{H} (250MHz, CDCl₃) inter alia 1.26 (15H, mult, PMe + OCH₂CH₃) 1.98 (4H, br. mult, PCH₂+PCH₂CH₂) 2.62-2.91 (18H, m, together 3 x P.CH₂N and Ring CH₂s), 3.48-3.75 (2H, mult, broad, CH₂NHCO), 3.97-4.27 (6H, m, together 3 x P.O. CH₂Me), 7.41-7.49 (3H, m, aromatic C₆H₂H₎ and 8.05 br (1H, s, NHCOPh), m/z (DCI, NH₃) 638 (M⁺+2), 637 (M⁺+1) 545, 256 and 109.

### Preparation of the hydrochloride salt of a salt of a compound of formula (1b) where R¹ is -CH₂P(O)(OH)(CH₂)₃NH₂ and R² and R³ is each -CH₂P(O)(OH)CH₃

A solution of Intermediate 8 (0.02g) in 6M -HCl (10cm³) was heated at 140°C for 48 hours to afford complete hydrolysis (as seen by Hnmr). The cooled solution was washed firstly with CH₂Cl₂ (2x) and than diethyl ether (2x) before evaporation under reduced pressure to give the title hydrochloride as a glassy foam (0.011g).

δ_{H} (D₂O) 1.36-1.50 (6H, mult of doublets, P-CH₃), 1.75-1.85 (4H, mult, CH₂PO + CH₂CH₂PO), 3.01 (2H, mult, CH₂NH₃+) 3.10-3.50 (18H, CH₂N+PCH₂N, mult) m/z (f.a.b., glyerol) 449 (M⁺+1), 448 (M+)

### Intermediate 10

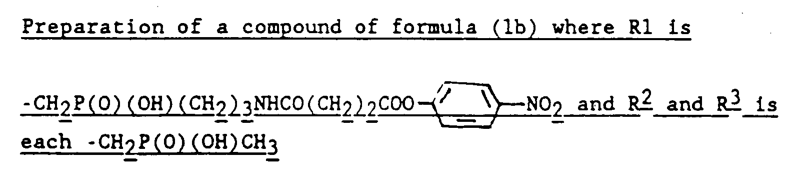

The title nitrophenyl ester was prepared using Intermediate 9 and the di-4-nitrophenyl ester succinic acid as described for the preparation of Intermediate 6.

### Intermediates 11 and 12

### Preparation of the ⁹⁰Y complexes of Intermediate 6 and 10

To a solution of either Intermediate 6 (5»∂m³) or Intermediate 10 (5»∂m²) in tetramethylammonium morpholinoethanesulphate (MES) buffer (0.1M, pH, 6.8, 90»∂m³) at 37°C was added 5»∂Ci of ⁹⁰Y (5»∂m³ of an aqueous solution of the trichloride) to produce the labelled products Intermediates 11 and 12. After 0.5h each mixture containing either Intermediate 11 or 12 was analysed by HPLC (AX 300: 0.2M NH₄OAc: 10% CH₃CN) with radiometric detection (LKB radiation detector) following quenching of the labelling reaction by addition of a 500 fold excess of diethylenetriaminepentaacetic acid (DTPA). Radiolabelling yields of 82% were determined (hplc radiometry integrating the ⁹⁰Y-ligand peak (4.5 mins) against ⁹⁰Y-DTPA (15 mins). After maintaining the complex at this pH at 298K in the presence of a 500 fold excess of DTPA, no change in the relative concentration of complex was deserved at 24, and 72h.

### Example

The labelled products, Intermediates 11 and 12 were each coupled to the antibody B72.3 using the following procedure.

B72.3 monoclonal antibody [Colcher, D. et al Proc. Nat. Acad. Sci. USA (1981), 78, 3199; 3.75mg previously modified with Traut's reagent] in 0.1M phosphate buffer (containing 2mM ethylenediaminetetraacetic acid; pH8.0; 110»l) was added to either Intermediate 11 or 12 (25»l) and the mixture was incubated at 37°C for 90 minutes then purified by PD-10 gel filtration chromatography to yield the desired labelled conjugate products.

## Claims

1. A conjugate compound comprising a compound of general formula (1) 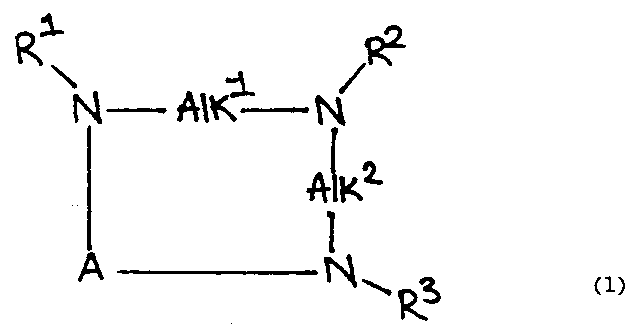 wherein
A is a group -Alk³- or -Alk³N(R⁴)Alk⁴-;
Alk¹, Alk², Alk³ and Alk⁴ which may be the same or different is each a C₁₋₄alkylene chain optionally substituted by one or more C₁₋₆alkyl groups;
and R¹, R², R³ and R⁴, which may be the same or different, is each a hydrogen atom or a group -AlkR⁵ where Alk is an optionally substituted straight or branched C₁₋₆ alkyl group and R⁵ is a hydrogen atom or a -CO₂H, -CONR⁶R⁷ (where R⁶ and R⁷, which may be the same or different is each a hydrogen atom or an alkyl group) or -P(X¹)(X²R⁸)L group where X¹ and X² is each an oxygen or sulphur atom, R⁸ is a hydrogen atom or an alkyl group and L is an aliphatic, aromatic, or heteroaromatic group or a linker group with the proviso that at least one of R¹, R², R³ and R⁴ is a group AlkP(X¹)(X²R⁸)L where L is a linker group; and protected derivatives and metal complexes and/or salts thereof, coupled to a protein, peptide or carbohydrate.

2. A conjugate according to Claim 1 wherein the compound of formula (1) or a metal complex or salt thereof is coupled to an antibody.

3. A conjugate according to Claims 1 or 2 wherein the compound of formula (1) is a compound of formula (1a): 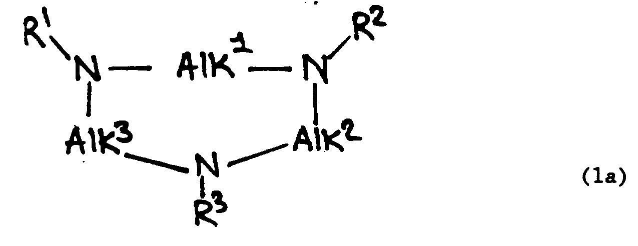 wherein R¹, R², R³, Alk¹, Alk² and Alk³ are as defined for formula (1) with the proviso that at least one of R¹, R² and R³ is a group AlkP(X¹)(X²R⁸)L where L is a linker group; and metal complexes and/or salts thereof.

4. A conjugate according to Claim 3 wherein the compound of formula (1a) is a compound of formula (1b): 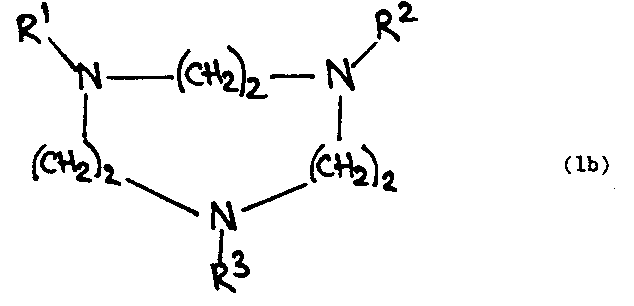 wherein R¹, R², and R³ are as defined for formula (1a) and metal complexes and/or salts thereof.

5. A conjugate according to Claim 4 wherein at least one of R¹, R² and R³ in formula (1b) is a group -AlkP(O)(OR⁸)L.

6. A conjugate according to Claim 5 wherein AlkP(O)(OR⁸)L is -CH₂P(O)(OH)L.

7. A conjugate according to Claims 3-6 wherein the compound of formulae (1a) or (1b) is complexed with indium, yttrium or gadolinium.

8. A conjugate according to Claims 1 or 2 wherein the compound of formula (1) is a compound of formula (1c) 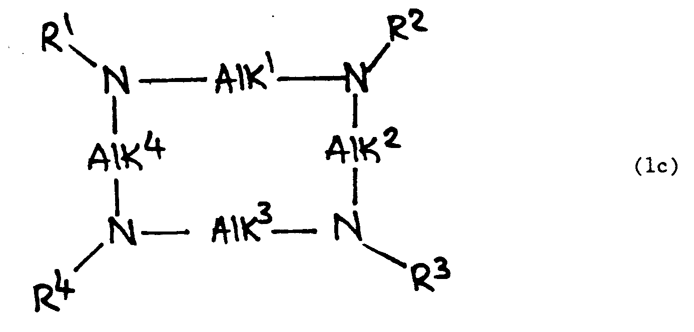 wherein R¹, R², R³, R⁴, Alk¹, Alk², Alk³ and Alk⁴ are as defined for formula (1) with the proviso that at least one of R¹, R², R³ and R⁴ is a group AlkP(X¹)(X²R⁸)L where L is a linker group; and protected derivatives and metal complexes and/or salts thereof.

9. A conjugate according to Claim 8 wherein the compound of formula (1c) is a compound of formula (1d). 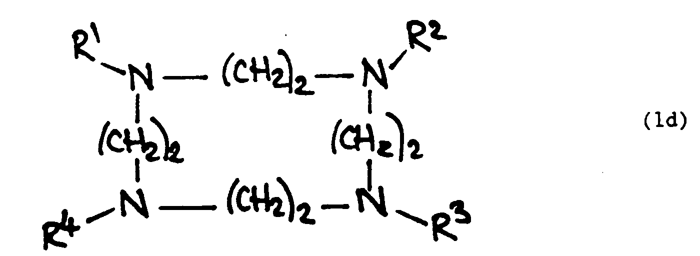 wherein R¹, R², R³ and R⁴ are as defined for formula (1c); and and metal complexes and/or salts thereof.

10. A conjugate according to Claim 9 wherein at least one of R¹, R², R³ and R⁴ in formula (1d) is -AlkP(O)(OR⁸)L.

11. A conjugate according to Claim 10 wherein -AlkP(O)(OR⁸)L is -CH₂P(O)(OH)L.

12. A conjugate according to Claims 8 - 10 wherein the compound of formulae (1c) or (1b) is complexes with yttrium or gadolinium.

## Patentansprüche

1. Konjugatverbindung, umfassend eine Verbindung mit der allgemeinen Formel (1) 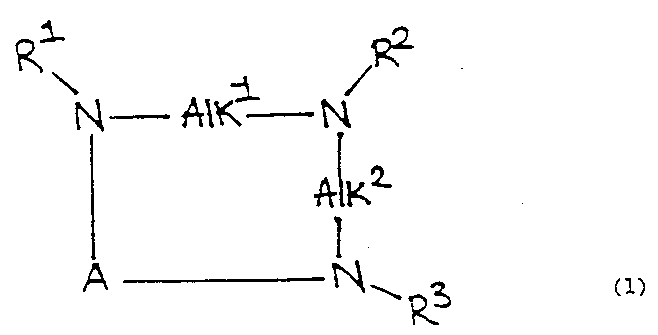 wobei:
A eine Gruppe -Alk³- oder -Alk³N(R⁴)Alk⁴- ist;
Alk¹, Alk², Alk³ und Alk⁴, welche gleich oder verschieden sein können, jeweils eine C₁₋₄Alkylenkette sind, die gegebenenfalls mit einer oder mehreren C₁₋₆Alkylgruppen substituiert ist;
und R¹, R², R³ und R⁴, welche gleich oder verschieden sein können, jeweils ein Wasserstoffatom oder eine Gruppe -AlkR⁵ sind, wobei Alk eine gegebenenfalls substituierte, gerade oder verzweigte C₁₋₆Alkylgruppe ist und R⁵ ein Wasserstoffatom oder eine Gruppe -CO₂H, -CONR⁶R⁷ (wobei R⁶ und R⁷, welche gleich oder verschieden sein können, jeweils ein Wasserstoffatom oder eine Alkylgruppe sind) oder -P(X¹)(X²R⁸)L ist, wobei X¹ und X² jeweils ein Sauerstoff- oder ein Schwefelatom sind, R⁸ ein Wasserstoffatom oder eine Alkylgruppe ist, und L eine aliphatische, aromatische oder heteroaromatische Gruppe oder eine Verbindungsgruppe ist, mit der Maßgabe, daß mindestens eine von R¹, R², R³ und R⁴ eine Gruppe AlkP(X¹)(X²R⁸)L ist, wobei L eine Verbindungsguppe ist;
und geschützte Derivate und Metallkomplexe und/oder Salze davon, die an ein Protein, ein Peptid oder ein Kohlehydrat gekoppelt sind.

2. Konjugat nach Anspruch 1, wobei die Verbindung der Formel (1) oder ein Metallkomplex oder ein Salz davon an einen Antikörper gekoppelt ist.

3. Konjugat nach den Ansprüchen 1 oder 2, wobei die Verbindung der Formel (1) eine Verbindung der Formel (1a): 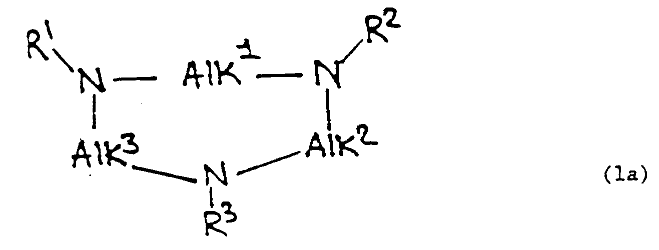 ist, wobei R¹, R², R³, Alk¹, Alk² und Alk³ wie für die Formel (1) definiert sind, mit der Maßgabe, daß mindestens eine von R¹, R² und R³ eine Gruppe AlkP(X¹)(X²R⁸)L ist, wobei L eine Verbindungsguppe ist; und Metallkomplexe und/oder Salze davon.

4. Konjugat nach Anspruch 3, wobei die Verbindung der Formel (1a) eine Verbindung der Formel (1b): 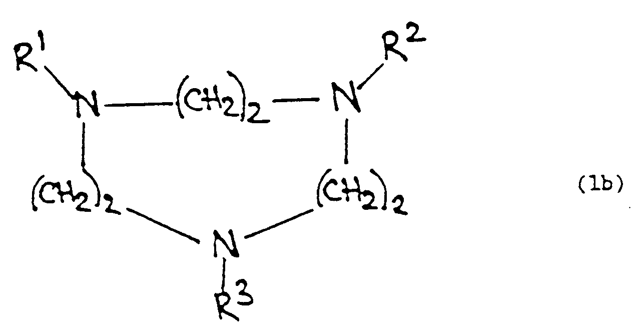 ist, wobei R¹, R² und R³ wie für die Formel (1a) definiert sind, und Metallkomplexe und/oder Salze davon.

5. Konjugat nach Anspruch 4, wobei mindestens eine von R¹, R² und R³ in der Formel (1b) eine Gruppe -AlkP(O)(OR⁸)L ist.

6. Konjugat nach Anspruch 5, wobei AlkP(O)(OR⁸)L -CH₂P(O)(OH)L ist.

7. Konjugat nach den Ansprüchen 3 - 6, wobei die Verbindung der Formeln (1a) oder (1b) mit Indium, Yttrium oder Gadolinium komplexiert ist.

8. Konjugat nach den Ansprüchen 1 oder 2, wobei die Verbindung der Formel (1) eine Verbindung der Formel (1c): 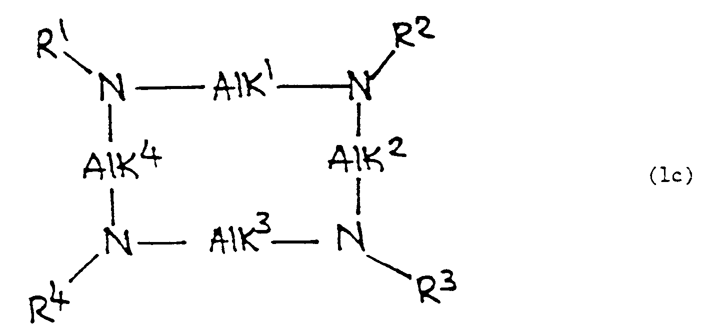 ist, wobei R¹, R², R³, R⁴, Alk¹, Alk², Alk³ und Alk⁴ wie für die Formel (1) definiert sind, mit der Maßgabe, daß mindestens eine von R¹, R², R³ und R⁴ eine Gruppe AlkP(X¹)(X²R⁸)L ist, wobei L eine Verbindungsguppe ist; und geschützte Derivate und Metallkomplexe und/oder Salze davon.

9. Konjugat nach Anspruch 8, wobei die Verbindung der Formel (1c) eine Verbindung der Formel (1d): 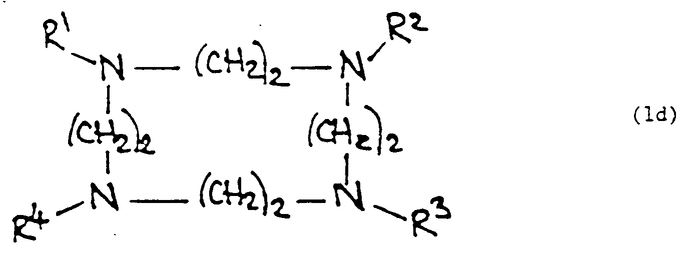 ist, wobei R¹, R², R³ und R⁴ wie für die Formel (1c) definiert sind; und Metallkomplexe und/oder Salze davon.

10. Konjugat nach Anspruch 9, wobei mindestens eine von R¹, R², R³ und R⁴ in der Formel (1d) -AlkP(O)(OR⁸)L ist.

11. Konjugat nach Anspruch 10, wobei -AlkP(O)(OR⁸)L -CH₂P(O)(OH)L ist.

12. Konjugat nach den Ansprüchen 8 - 10, wobei die Verbindungen der Formeln (1c) oder (1b) Komplexe mit Yttrium oder Gadolinium sind.

## Revendications

1. Composé conjugué, comprenant un composé de formule générale (1) 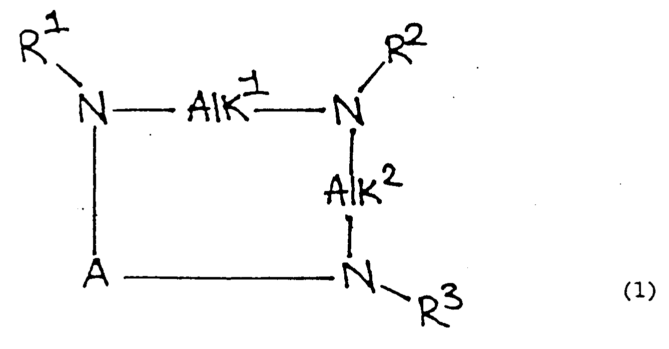 dans laquelle
A est un groupe -Alk³- ou -Alk³N(R⁴)Alk⁴-;
Alk¹, Alk², Alk³ et Alk⁴, qui peuvent être identiques ou différents, sont chacun une chaîne alkylène en C₁-C₄, éventuellement substituée par un ou plusieurs groupes alkyle en C₁-C₆;
et R¹, R², R³ et R⁴, qui peuvent être identiques ou différents, sont chacun un atome d'hydrogène ou un groupe -AlkR⁵, où Alk est un groupe alkyle en C₁-C₆, linéaire ou ramifié, éventuellement substitué, et R⁵ est un atome d'hydrogène ou un groupe -CO₂H, -CONR⁶R⁷ (où R⁶ et R⁷, qui peuvent être identiques ou différents, sont chacun un atome d'hydrogène ou un groupe alkyle) ou -P(X¹)(X²R⁸)L, dans lequel X¹ et X² sont chacun un atome d'oxygène ou de soufre, R⁸ est un atome d'hydrogène ou un groupe alkyle et L est un groupe aliphatique, aromatique ou hétéroaromatique, ou un groupe de liaison, à condition que l'un au moins des radicaux R¹, R², R³ et R⁴ soit un groupe AlkP(X¹)(X²R⁸)L, dans lequel L est un groupe de liaison; et les dérivés protégés et les complexes métalliques et/ou les sels de ce composé, couplés à une protéine, à un peptide ou à un sucre.

2. Conjugué selon la revendication 1, dans lequel le composé de formule (1) ou un complexe métallique ou un sel de ce composé est couplé à un anticorps.

3. Conjugué selon la revendication 1 ou 2, dans lequel le composé de formule (1) est un composé de formule (1a): 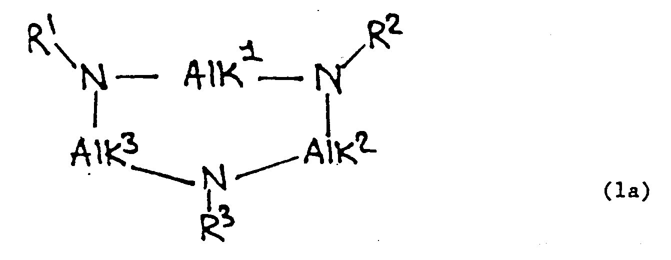 dans laquelle R¹, R², R³, Alk¹, Alk² et Alk³ sont tels que définis pour la formule (1), à condition que l'un au moins des radicaux R¹, R² et R³ soit un groupe AlkP(X¹)(X²R⁸)L, dans lequel L est un groupe de liaison; et les complexes métalliques et/ou les sels de ce composé.

4. Conjugué selon la revendication 3, dans lequel le composé de formule (1a) est un composé de formule (1b): 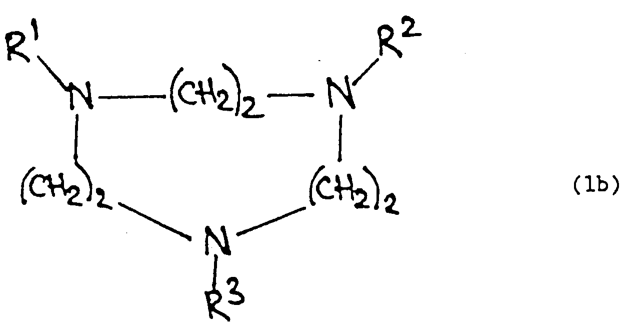 dans laquelle R¹, R² et R³ sont tels que définis pour la formule (1a), et les complexes métalliques et/ou les sels de ce composé.

5. Conjugué selon la revendication 4, dans lequel l'un au moins des radicaux R¹, R² et R³, dans la formule (1b), est un groupe -AlkP(O)(OR⁸)L.

6. Conjugué selon la revendication 5, dans lequel -AlkP(O)(OR⁸)L est -CH₂P(O)(OH)L.

7. Conjugué selon les revendications 3-6, dans lequel le composé de formule (1a) ou (1b) est complexé avec de l'indium, de l'yttrium ou du gadolinium.

8. Conjugué selon la revendication 1 ou 2, dans lequel le composé de formule (1) est un composé de formule (1c) 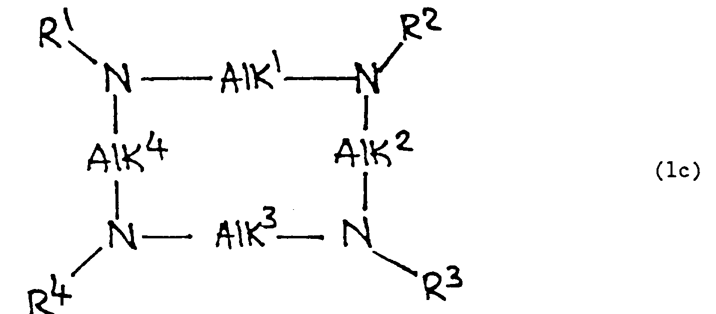 dans laquelle R¹, R², R³, R⁴, Alk¹, Alk², Alk³ et Alk⁴ sont tels que définis pour la formule (1), à condition que l'un au moins des radicaux R¹, R², R³ et R₄ soit un groupe AlkP(X¹)(X²R⁸)L, dans lequel Y est un groupe de liaison; et les dérivés protégés et les complexes métalliques et/ou les sels de ce composé.

9. Conjugué selon la revendication 8, dans lequel le composé de formule (1c) est un composé de formule (1d): 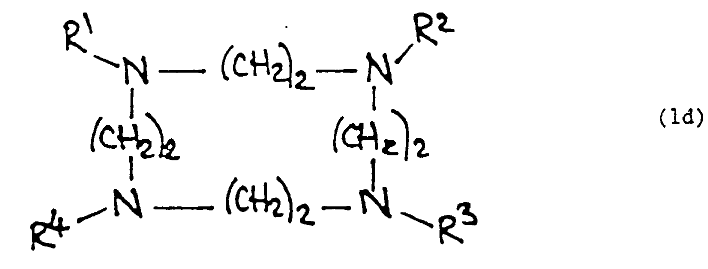 dans laquelle R¹, R², R³ et R⁴ sont tels que définis pour la formule (1c); et les complexes métalliques et/ou les sels de ce composé.

10. Conjugué selon la revendication 9, dans lequel l'un au moins des radicaux R¹, R², R³ et R⁴, dans la formule (1d), est un groupe -AlkP(O)(OR⁸)Y.

11. Conjugué selon la revendication 10, dans lequel -AlkP(O)(OR⁸)L est -CH₂P(O)(OH)L.

12. Conjugué selon les revendications 8-10, dans lequel le composé de formule (1c) ou (1d) est complexé avec de l'yttrium ou du gadolinium.
